# EUROPÄISCHE PATENTSCHRIFT

(11) **EP 1 401 871 B1**
(45) Veröffentlichungstag und Bekanntmachung des Hinweises auf die Patenterteilung: **23.03.2005**
(21) Anmeldenummer: 02782453.1
(22) Anmeldetag: 03.07.2002
(51) Int. Cl.: C07K 16/18, A61K 39/395, C12N 15/13

(54) **IMMUNOLOGISCHE BINDUNGSMOLEKÜLE, DIE DIE SYNZYTIALE FUSION VON TROPHOBLASTZELLEN HEMMEN**
IMMUNOLOGICAL BINDING MOLECULES INHIBITING THE SYNCYTIAL FUSION OF TROPHOBLAST CELLS
MOLECULES DE LIAISON IMMUNOLOGIQUES INHIBANT LA FUSION SYNCYTIALE DE CELLULES DU TROPHOBLASTE

(30) Priorität: 04.07.2001 EP 01116199
(43) Veröffentlichungstag der Anmeldung: 31.03.2004
(73) Patentinhaber: AplaGen GmbH, 52499 Baesweiler (DE)
(72) Erfinder: FRANK, Hans-Georg, NL-6462 EL Kerkrade (NL); KAUFMANN, Peter, NL-6291 CX Vaals (NL); SCHMITZ, Ulrike, 52070 Aachen (DE)
(74) Vertreter: Meyers, Hans-Wilhelm, Dr.Dipl.-Chem.
(86) Internationale Anmeldenummer: PCT/EP2002/007359
(87) Internationale Veröffentlichungsnummer: WO 2003/004533

(56) Entgegenhaltungen:
- WO-A-01/49710
- WO-A-93/06857
- MI SHA ET AL: "Syncytin is a captive retroviral envelope protein involved in human placental morphogenesis." NATURE (LONDON), Bd. 403, Nr. 6771, 17. Februar 2000 (2000-02-17), Seiten 785-789, XP002184567 ISSN: 0028-0836 in der Anmeldung erwähnt
- HUPPERTZ B ET AL: "Apoptosis and syncytial fusion in human placental trophoblast and skeletal muscle" INT REV CYTOL, Bd. 205, 2001, Seiten 215-253, XP001034137
- POETGENS A J G ET AL: "MECHANISMS OF SYNCYTIAL FUSION: A REVIEW" PLACENTA, LONDON, GB, Bd. 23, Nr. 16, SUPPL A, April 2002 (2002-04), Seiten S107-S113, XP001096202

## Beschreibung

Gegenstand der vorliegenden Erfindung sind Proteine, die die synzytiale Fusion menschlicher Trophoblastzellen hemmen, Arzneimittel enthaltend diese Proteine sowie deren Verwendung zur Herstellung eines Arzneimittels zur Kontrazeption bei Säugern.

Die Hemmung der synzytialen Fusion stellt ein Wirkprinzip dar, das zur Kontrazeption benutzt werden kann. Der Begriff der Kontrazeption wird in dieser Schrift unter klinisch-praktischen Gesichtspunkten so definiert, dass trotz geschlechtlichen Kontaktes eine zeitgerechte monatliche Regelblutung einsetzt und keine Schwangerschaft beginnt. Diese allgemeine Definition gilt auch für die meisten der heute klinisch gebräuchlichen Kontrazeptiva. Die Kontrazeption bei Mensch und anderen Säugetieren kann durch Anwendung einer Reihe von Prinzipien bzw. Substanzen erreicht werden. Bei den bekannten Verfahren wird entweder durch mechanische oder chemische Mittel die Verschmelzung von Eizelle und Samenzelle verhindert. Die Samenzelle kann z.B. durch Benutzung von Kondomen, intrauterin verbrachten Implantaten oder spermiziden Substanzen inaktiviert bzw. an der Befruchtung gehindert werden. Des weiteren besteht die Möglichkeit, die Reifung der Eizelle im mütterlichen Ovar zu behindern, was durch hormonelle Methoden möglich ist. Solche Verfahren sind dem Fachmann bekannt.

Eine große Gruppe der heute gebräuchlichen hormonellen Kontrazeptiva ("Pille") beeinflusst die Schleimhaut der Gebärmutter (das Endometrium) so, dass eine Einnistung der Blastozyste nicht mehr möglich ist, weil die Rezeptivität des Endometriums nicht mehr gegeben ist. Diese endokrinen Verfahren der Kontrazeption sind mit dem Problem behaftet, dass eine zeit- und dosisgenaue Einnahme der Hormone gewährleistet werden muss, um die erwünschte Wirkung zu erzielen. Da Hormone selbst nur eine kurze - nach Stunden bis längstens Tagen - bemessene Halbwertszeit haben, ist eine erfolgreiche Kontrazeption mit dieser Methode nur bei täglicher Einnahme oder durch die Verabreichung von Depotpräparaten möglich. Alle diese Lösungen sind mit dem Problem behaftet, dass sie die zyklische endokrine Homöostase des weiblichen Organismus stören, bzw. unterbinden. Weil das Endometrium ein Bestandteil des mütterlichen Organismus ist, ist der mütterliche Organismus auch der bevorzugte Ort des Auftretens unerwünschter Nebenwirkungen.

Wünschenswert sind daher Verfahren und Substanzen, die
a) an der implantierenden Blastozyste ihren Wirkort haben und damit die Nebenwirkungen im mütterlichen Organismus zusätzlich minimieren können.
b) Nicht mit der endokrinen Autoregulation des weiblichen Organismus interferieren.

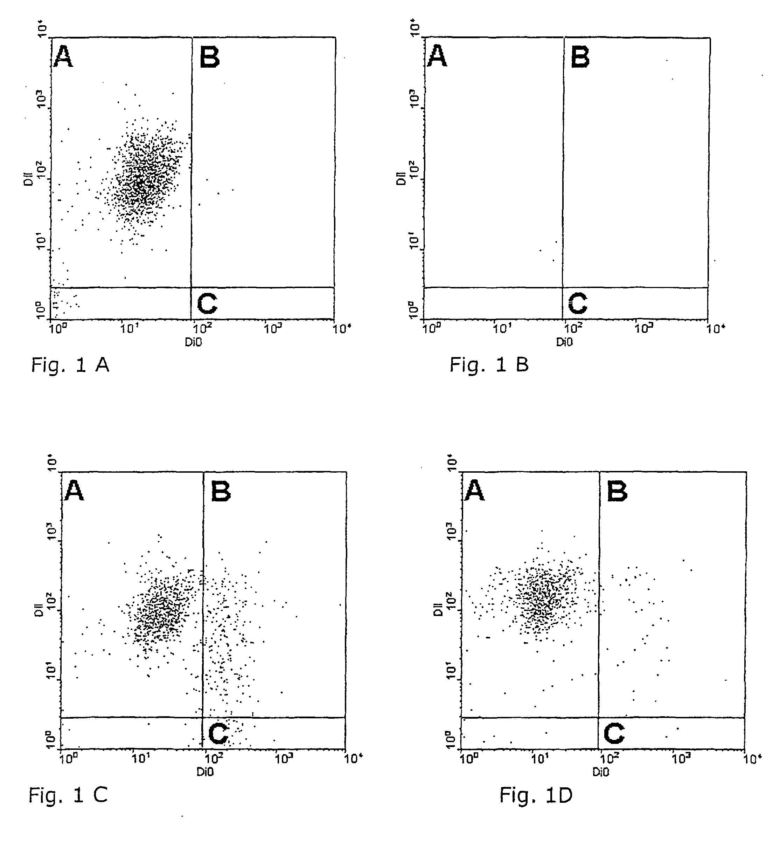

   Figur 1 zeigt ein Beispiel einer flow-cytometrischen Analyse im Rahmen eines Fusionsassays. Im Quadranten A wird dabei die Fluoreszenz von DiI (alleine) dargestellt, während in Quadrant C die Fluoreszenz von DiO (alleine) dargestellt wird. Im Quadranten B werden Zellen sichtbar, die fusionsbedingte Doppelfluoreszenz aufweisen.
   Figur 1A,B zeigen Kontrollen, in denen die Zellen mit jeweils nur einem Fluoreszenzfarbstoff gefärbt und unter den Bedingungen des Assay inkubiert wurden. In Figur 1A wurde nur mit DiI, in Figur 1B nur mit DiO gefärbt. Die Messwerte für DiI sind im DiO-spezifischen Messkanal gleich null; daher fallen die Punkte dort mit der X-Achse zusammen.
   Figur 1C,D zeigen die Fusionsassays des Klones PHSK04. Figur 1C zeigt die Rate der Fusion der Zellen unter Kontrollbedingungen (in Gegenwart eines unspezifischen Proteins, im selben Expressionssystem exprimiert und gereinigt) und Figur 1D zeigt die Fusionsrate in Gegenwart von 100nM PHSK04-Protein.

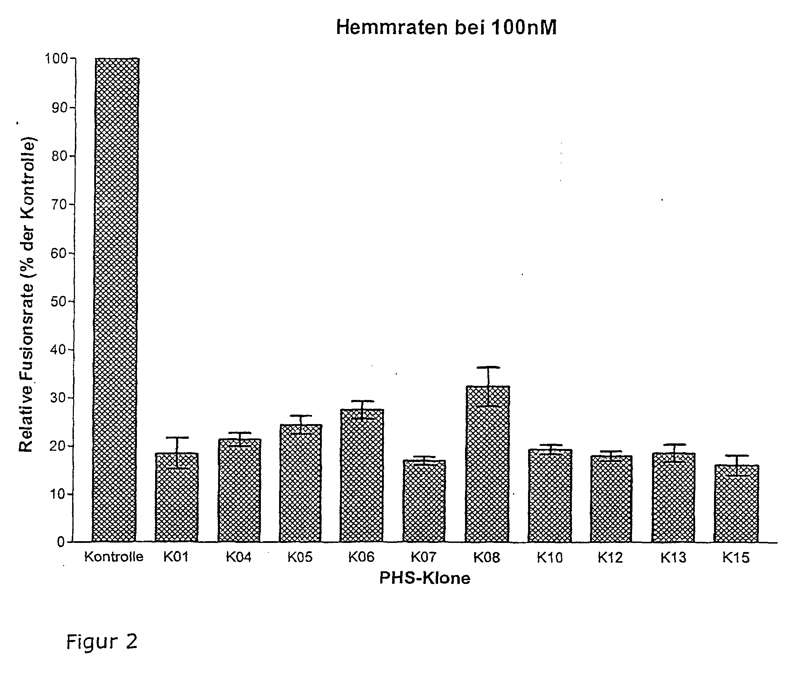

   Figur 2 zeigt die relativen Hemmraten der Beispielklone im Fusionsassay im Vergleich zu einer Kontrolle (Calmodulin, im selben Expressionssystem exprimiert und gereinigt). Dargestellt sind Mittelwerte aus drei Bestimmungen.

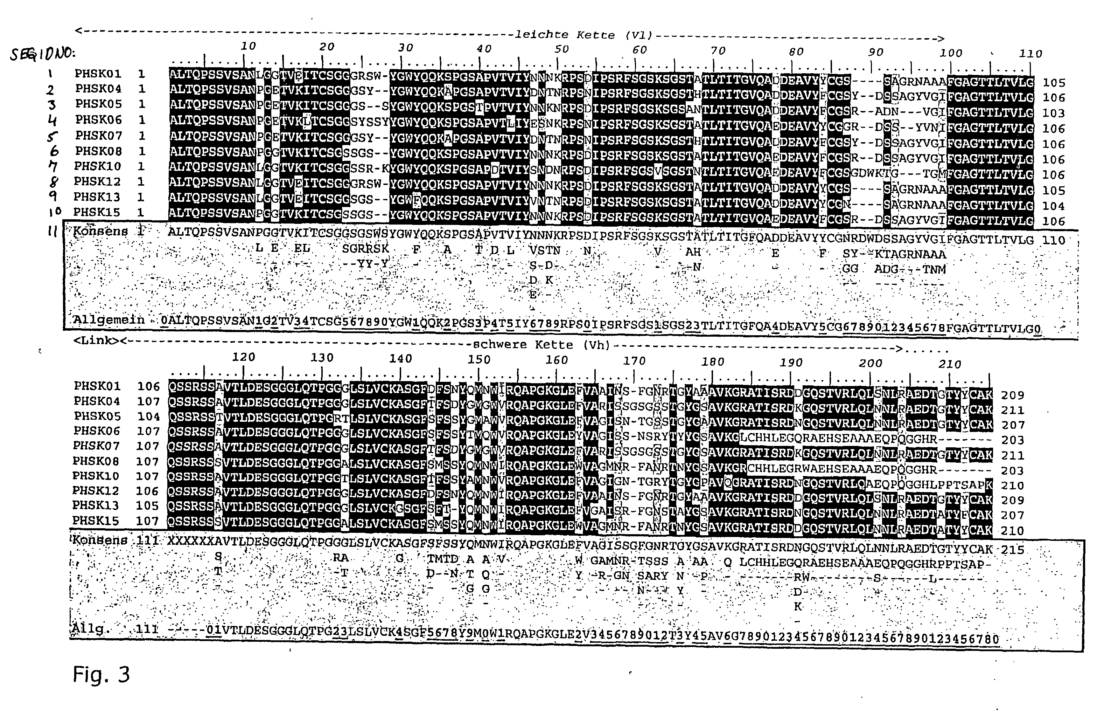

   Figur 3 zeigt die Konsensussequenz für die erfindungsgemäß bevorzugten Proteine mit der Sq. ID Nr. 1 bis 10. Linker ist in der hier dargestellten Beispielausführung die Sequenz QSSRSS. Die Konsensussequenzen für VI und Vh sind grau unterlegt unter den Einzelsequenzen der Klone im Einbuchstabencode dargestellt. Die Konsensussequenz selbst ist dabei horizontal zu lesen, während für jede Position vertikal bevorzugte Alternativ-aminosäuren im Einbuchstabencode aufgeführt sind. Ein Bindestrich an dieser Stelle bedeutet, dass die entsprechende Position im Vergleich zur Konsensussequenz auch fehlen kann. Ein X bedeutet, dass an dieser Stelle kein Teil der Konsensussequenzen für VI respektive Vh vorliegt.

In Gegenwart von PHSK04-Protein ist die Fusionsrate erniedrigt.

Das der Erfindung zugrunde liegende Problem wird gelöst durch ein Protein, das mit VI oder Vh des Huhns zu mindestens 80 % homolog ist und eine synzytiale Fusion hemmt sowie Fragmente mit mindestens 12 zusammenhängenden Aminosäuren aus der Sequenz der VI oder VH des Huhns. In bevorzugten Ausführungsformen ist das erfindungsgemäße Protein zu mindestens 85 oder mindestens 90% homolog mit VI oder Vh des Huhns. In bevorzugten Ausführungsformen weisen die erfindungsgemäßen Fragmente mindestens 20 oder 30 zusammenhängende Aminosäuren aus der Sequenz der VI oder VH des Huhns auf.

Vorzugsweise besitzt das erfindungsgemäße Protein VI die nachfolgende Aminosäuresequenz: wobei
- X⁰ =: eine unsubstituierte, mono oder disubstituierte Aminogruppe oder eine oder mehrere Aminosäuren bedeutet,
- X¹ =: P oder L oder eine Deletion,
- X² =: G oder E oder eine Deletion,
- X³ =: K oder E oder eine Deletion,
- X⁴ =: I oder L oder eine Deletion,
- X⁵ =: G oder S oder eine Deletion,
- X⁶ =: S oder G oder eine Deletion,
- X⁷ =: G oder R oder Y oder eine Deletion,
- X⁸ =: S oder R oder Y oder eine Deletion,
- X⁹ =: W oder S oder eine Deletion,
- X¹⁰ =: S oder K oder Y oder eine Deletion,
- X¹¹ =: Y oder F oder eine Deletion,
- X¹² =: S oder A oder eine Deletion,
- X¹³ =: A oder T oder eine Deletion,
- X¹⁴ =: V oder D oder eine Deletion,
- X¹⁵ =: V oder L oder eine Deletion,
- X¹⁶ =: N oder V oder S oder D oder E oder eine Deletion,
- X¹⁷ =: N oder S oder eine Deletion,
- X¹⁸ =: N oder T oder D oder K oder eine Deletion,
- X¹⁹ =: K oder N oder eine Deletion,
- X²⁰ =: D oder N oder eine Deletion,
- X²¹ =: K oder V oder eine Deletion,
- X²² =: T oder A oder eine Deletion,
- X²³ =: A oder H oder N oder eine Deletion,
- X²⁴ =: D oder E oder eine Deletion,
- X²⁵ =: Y oder F oder eine Deletion,
- X²⁶ =: N oder S oder G oder eine Deletion,
- x²⁷ =: R oder Y oder G oder eine Deletion,
- X²⁸ =: D oder eine Deletion,
- X²⁹ =: W oder eine Deletion,
- X³⁰ =: D oder K oder A oder eine Deletion,
- X³¹ =: S oder T oder D oder eine Deletion,
- X³² =: S oder A oder G oder eine Deletion,
- X³³ =: A oder G oder eine Deletion,
- X³⁴ =: G oder R oder eine Deletion,
- X³⁵ =: Y oder N oder eine Deletion,
- X³⁶ =: V oder A oder T oder eine Deletion,
- X³⁷ =: G oder A oder N oder eine Deletion,
- X³⁸ =: I oder A oder M oder eine Deletion ist und
- X³⁹ =: eine Carboxylgruppe, ein Carboxylderivat oder eine oder mehrere Aminosäuren bedeutet
und wobei konservative Austausche jeder beliebigen Aminosäure untereinander zulässig sind, sofern die Hemmung der synzytialen Fusion erhalten bleibt.

In einer weiteren bevorzugten Ausführungsform weist das erfindungsgemäße Protein in Vh die nachfolgende Sequenz auf: wobei
- X⁰ =: eine unsubstituierte, mono oder disubstituierte Aminogruppe oder eine oder mehrere Aminosäuren bedeutet,
- X¹ =: A oder S oder T oder eine Deletion,
- X² =: G oder R oder eine Deletion,
- X³ =: G oder A oder T oder eine Deletion,
- X⁴ =: A oder G oder eine Deletion,
- X⁵ =: S oder T oder D oder eine Deletion,
- X⁶ =: F oder M oder eine Deletion,
- X⁷ =: S oder T oder eine Deletion,
- X⁸ =: S oder D oder N oder eine Deletion,
- X⁹ =: G oder Q oder A oder T oder eine Deletion,
- X¹⁰ =: N oder A oder Q oder G oder eine Deletion,
- X¹¹ =: I oder V oder eine Deletion,
- X¹² =: F oder W oder Y oder eine Deletion,
- X¹³ =: A oder G oder eine Deletion,
- X¹⁴ =: G oder A oder R oder eine Deletion,
- X¹⁵ =: I oder M oder eine Deletion,
- X¹⁶ =: S oder N oder G oder eine Deletion,
- X¹⁷ =: S oder R oder N oder eine Deletion,
- X¹⁸ =: G oder eine Deletion,
- X¹⁹ =: F oder T oder S oder N oder eine Deletion,
- X²⁰ =: G oder S oder A oder eine Deletion,
- X²¹ =: N oder S oder R oder eine Deletion,
- X²² =: R oder S oder Y oder eine Deletion,
- X²³ =: G oder A oder N oder Y oder eine Deletion,
- X²⁴ =: G oder A oder eine Deletion,
- X²⁵ =: S oder A oder P oder eine Deletion,
- X²⁶ =: K oder Q oder eine Deletion,
- X²⁷ =: R oder L oder eine Deletion,
- X²⁸ =: A oder C oder eine Deletion,
- X²⁹ =: T oder H oder eine Deletion,
- X³⁰ =: I oder H oder eine Deletion,
- X³¹ =: S oder L oder eine Deletion,
- X³² =: R oder E oder eine Deletion,
- X³³ =: D oder G oder eine Deletion,
- X³⁴ =: N oder Q oder R oder D oder K oder eine Deletion,
- X³⁵ =: G oder R oder W oder eine Deletion,
- X³⁶ =: Q oder A oder eine Deletion,
- X³⁷ =: S oder E oder eine Deletion,
- X³⁸ =: T oder H oder eine Deletion,
- X³⁹ =: V oder S oder eine Deletion,
- X⁴⁰ =: R oder E oder eine Deletion,
- X⁴¹ =: L oder A oder eine Deletion,
- X⁴² =: Q oder A oder eine Deletion,
- X⁴³ =: L oder A oder eine Deletion,
- X⁴⁴ =: N oder E oder S oder eine Deletion,
- X⁴⁵ =: N oder Q oder eine Deletion,
- X⁴⁶ =: L oder P oder eine Deletion,
- X⁴⁷ =: R oder Q oder eine Deletion,
- X⁴⁸ =: A oder G oder eine Deletion,
- X⁴⁹ =: E oder G oder eine Deletion,
- X⁵⁰ =: D oder H oder eine Deletion,
- X⁵¹ =: T oder R oder L oder eine Deletion,
- X⁵² =: G oder P oder eine Deletion,
- X⁵³ =: T oder P oder eine Deletion,
- X⁵⁴ =: Y oder T oder eine Deletion,
- X⁵⁵ =: Y oder S oder eine Deletion,
- X⁵⁶ =: C oder A oder eine Deletion,
- X⁵⁷ =: A oder P oder eine Deletion,
- X⁵⁸ =: K oder eine Deletion ist,
- X⁵⁹ =: eine Carboxylgruppe, ein Carboxylderivat oder eine o der mehrere Aminosäuren bedeutet
und wobei konservative Austausche jeder beliebigen Aminosäure untereinander zulässig sind, sofern die Hemmung der synzytialen Fusion erhalten bleibt.

Unter konservativen Austauschen sind erfindungsgemäß insbesondere solche zu verstehen, die Aminosäuren innerhalb der Gruppen der polaren, unpolaren, aromatischen, geladenen Aminosäuren austauschen. Zum Beispiel Glycin gegen Alanin oder Valin usw., Arginin oder Lysin gegen Histidin usw., Phenylalanin gegen Thyrosin usw.

Die erfindungsgemäßen Proteine können erfindungsgemäß miteinander über einen Linker verbunden sein. Der Linker ist vorzugsweise eine Peptidkette aus mindestens fünf beliebigen Aminosäuren. Dabei werden die Aminosäuren bevorzugt, die keine oder nur unerhebliche Wechselwirkung mit der VI oder Vh Kette bilden. Anstelle der Peptidkette kann der Linker auch aus organischen Verbindungen bestehen, die im wesentlichen aus einer Kette Kohlenstoffatomen, die auch Heteroatome aufweisen kann, gebildet werden. Die Länge des Linkers beträgt dann vorzugsweise mindestens neun Angström.

Die Anwesenheit von D-Aminosäuren in der Sequenz des erfindungsgemäßen Proteins kann vorteilhaft sein, da dies zur Verlangsamung des Abbaus führen kann.

Insbesondere werden die erfindungsgemäßen Proteine mit den Aminosäuresequenzen Seq ID Nr. 1 bis 10 bevorzugt.

Diese Proteine werden durch die Konsensussequenz mit der Sequenz Seq ID Nr 11, die ebenfalls Gegenstand der vorliegenden Erfindung ist, beschrieben.

Gegenstand der Erfindung sind auch Nucleinsäuren, insbesondere DNA oder RNA, codierend für ein erfindungsgemäßes Protein, sowie ein Plasmid oder Vektor, dass eine solche Nucleinsäure enthält.

Gegenstand der Erfindung sind auch Arzneimittel enthaltend mindestens eines der erfindungsgemäßen Proteine oder Nucleinsäuren.

Erfindungsgemäß beansprucht wird auch die Verwendung mindestens eines der erfindungsgemäßen Proteine oder Nucleinsäuren zur Herstellung eines Arzneimittels zur Kontrazeption bei Säugern. Das erfindungsgemäße Arzneimittel wird typischerweise in Mengen von 100 ng/kg bis 1000 µg/kg Körpergewicht verabreicht. Die Formulierung erfolgt vorzugsweise parenteral, durch Infusion durch intramuskuläre oder subcutane Injektion. Des weiteren sind mögliche Darreichungsformen ein Nasal-spray, Zäpfchen, vaginale Applikation z. B. über einen Tampon oder geeignete andere Darreichungsformen, die dem Fachmann auf dem Gebiet der Galenik an sich bekannt oder in einfachen Versuchen ermittelbar sind.

Die erfolgreiche Implantation der Blastozyste im Endometrium ist nicht nur von der Rezeptivität des Endometriums abhängig. Die Implantation ist nur möglich, wenn die Zellen in der äußeren Begrenzung der Blastozyste, die sogenannten Trophoblastzellen, miteinander verschmelzen und dabei ein Synzytium bilden. Die Bildung eines Synzytiums ist ein Vorgang, der in der Biologie des menschlichen Körpers nur an wenigen Stellen vorkommt. So ist dies bei der Entstehung von Skelettmuskelfasem und bei der Bildung von Osteoklasten bekannt.

Bei der synzytialen Fusion von Myoblasten oder bei der Entstehung von Osteoklasten sind Moleküle aus der Gruppe der ADAM ( a disintegrin and a metalloproteinase; für eine Übersicht siehe (Huppertz et al. 2001)) von besonderer Bedeutung, während der Trophoblast des Menschen ein Protein eines endogenen Retrovirus, das Syncytin (Mi et al. 2000), benutzt. Damit ist ein Ansatzpunkt gegeben, kontrazeptive Wirkung durch Hemmung der Fusion von Trophoblastzellen zu erzielen. Der Vorgang der Trophoblastfusion ist spezifisch für den Trophoblasten und seine Hemmung kann daher für die selektive, trophoblastspezifische Kontrazeption benutzt werden.

Funktionell sind die erfindungsgemäßen Proteine durch ihre Eigenschaft charakterisiert, dass sie die synzytiale Fusion von Trophoblastzellen zu hemmen vermögen. Sie sind verwendbar, um kontrazeptive Wirkung in weiblichen Säugetieren, insbesondere Nagern, Haustieren wie Hund und Katze sowie beim Menschen zu erzielen. Die Erfindung umfasst daher im wesentlichen folgende Bestandteile: Proteine, die vorzugsweise als immunologische Bindungsmoleküle vorliegen können, die
a) durch Homologievergleich als Abkömmlinge der Antikörper eines Nicht-Säugetieres, insbesondere des Huhnes, klassifiziert werden können. In besonderer Weise sind sie stofflich durch Benennung einer Aminosäure-Konsensussequenz charakterisiert;
b) funktionell durch die Eigenschaft charakterisiert sind, dass sie die synzytiale Fusion von Säugetier- Trophoblastzellen hemmen.

### Baueinheiten der immunologischen Bindungsmoleküle

Die erfindungsgemäßen Proteine können in einer bevorzugten Ausführungsform als immunologische Bindungsmoleküle vorliegen. Bei den immunologischen Bindungsmolekülen handelt es sich im hier beschriebenen Ausführungsbeispiel um rekombinante Moleküle. Die in den Beispielen ausgeführten immunologischen Bindungsmoleküle sind vom Typus des "single chain variable Fragment" Antikörpers (scFv). Ein solches Antikörpermolekül besteht nur aus den variablen Regionen der leichten (VI) und schweren Kette (Vh) eines Antikörpermoleküls die über eine kurze, rekombinant eingefügte Sequenz (Linker) miteinander verbunden sind. Ein scFv ist daher als eine minimalistische Ausprägungsform eines immunologischen Bindungsmoleküls zu verstehen. Die entscheidende. Bindungsinformation ist in den einzelnen variablen Regionen VI und Vh enthalten, nicht jedoch in der Art und Weise Ihrer Verknüpfung. Weitere Ausführungstypen der vorliegenden Erfindung betreffen den sogenannte Fab (antigenbindendes Fragment) Antikörper oder andere, beliebig konstruierte und ggf. auch nicht-rekombinante natürliche Antikörpermoleküle die wenigstens eine der beteiligten variablen Regionen in ihrer Sequenz aufweisen. In dem auch in den Beispielen dargestellten Ausführungstyp des scFv sind folgende Abfolgen der beteiligten Module denkbar:
VI - Linker - Vh
Vh - Linker - VI

Für andere Trägerkonstruktionen gilt ebenfalls, dass die Reihenfolge von VI und Vh in den Konstrukten nicht festgelegt ist, und dass das Konstrukt selbst nicht entscheidend zu den Bindungseigenschaften beiträgt. Unter Trägerkonstruktionen sind hier solche Moleküle zu verstehen, auf die scFv-Antikörper oder Teile dieser Antikörper montiert werden können, um sie in optimaler Weise ihren Bindungspartner finden zu lassen.

Die vorliegende Erfindung bezieht sich daher insbesondere auf alle immunologischen Bindungsmoleküle und ihre Derivate, deren spezifisch bindenden Regionen die unten aufgeführten Konsensussequenzen für Vh und VI aufweisen oder zu mindestens 80% sequenzidentisch mit der Konsensussequenz für jeweils VI oder Vh sind und die Fusion von Trophoblastzellen hemmen können. Ebenfalls ist es für die vorliegende Erfindung nicht wesentlich, ob bei der rekombinanten Expression der immunologischen Bindungsmoleküle aus technischen Gründen sogenannte Epitop-tags (z.B. HIS-tag zur besseren Reinigung oder myc-tag zum Einsatz von Sekundärantikörpern bei immunologischen Nachweisverfahren, etc.) eingebracht werden. Diese TAG's sind rein technischer Natur und nicht Bestandteil der hier zu schützenden Sequenzen.

Immunologische Bindungsmoleküle wie die hier beschriebenen können aus Hühnern gewonnen werden, indem das Immunrepertoire von Hühnern in geeigneter Form in molekularbiologische Bibliotheken überführt wird. Geeignete Techniken sind z.B. beschrieben in Barbas CF, Burton DR, Scott JK, Silverman GJ; Phage Display: A laboratory manual. CSHL Press, New York. Stehen solche Bibliotheken zur Verfügung, können die gewünschten Moleküle dadurch angereichert werden, dass anhand bekannter Techniken (z.B. beschrieben in Barbas *et al*., s.o.) solche Antikörper isoliert werden, die an die Oberfläche fusionsbereiter Trophoblastzellen binden. Anschliessend werden diese Antikörper auf der DNA-Ebene durch Vereinzelung kloniert und auf der DNA-Ebene sequenziert. Für die Kontrolle ihrer fusionshemmenden Wirkung müssen dann alle Antikörper im Fusionsassay getestet und charakterisiert werden. Die Sequenzen solcher aus stochastischen Bibliotheken isolierter fusionshemmender Antikörper werden durch die hier angegebenen Konsensussequenz mit hoher Wahrscheinlichkeit erfasst.

Alternativ kann Antiköerper-DNA bekannter Sequenz dadurch hergestellt werden, daß nach obigem Verfahren im Phage Display Hühnerantikörper kloniert und einzelne Klone zufällig ausgewählt und sequenziert werden (Andris-Widhopf et al.). Die Rahmensequenzen der Hühnerantikörper (Framework-sequences) sind hoch homolog und weichen wenig von den hier sequenzierten Vh bzw. VI Rahmensequenzen ab. Die Bindungsspezifität wird durch die "Complementarity-determining regions" (CDR) gewährleistet, die von Antikörper zu Antikörper sehr unterschiedlich sind. Durch gezielten Austausch einzelner Abschnitte, vor allen Dingen der CDR-Regionen, mit Hilfe bekannter molekularbiologischer Techniken lassen sich so auch Antikörper einer gewünschten Sequenz unter Abwandlung der klonierten Hühnersequenzen erstellen.

### Beispiele

### Antikörpermoleküle

Die im Beispiel ausgeführten scFv-Antikörper liegen - codiert in DNA -im Vektor pAC vor. Dieser Vektor ist ein für E.coli geeigneter Expressionsvektor, der mit einem fl-origin, einem durch Arabinose zu steuernden pBAD-Promotor sowie einem Gen besteht, das Ampicillinresistenz vermittelt. Die die immunologischen Bindungsmoleküle codierenden DNA-Sequenzen sind so in den Vektor eingebracht, dass Ihre Expression durch den pBAD Promotor kontrolliert wird. Ein im Vektor befindliches Signalpeptid dirigiert die exprimierten Proteine in das Periplasma von E. coli und ein 6xHIS-tag wird an die zu exprimierende Sequenz angehängt, um die Reinigung mittels Metall-Affinitätschromatographie zu ermöglichen. Alle Klone sind vor der Expression durch Sequenzierung verifiziert, so dass zweifelsfrei feststeht, dass die eingesetzten DNA-Sequenzen korrekt im Vektor eingebaut wurden.

Die Sequenzen können in jedem zur Expression von Proteinen geeigneten Expressionssystem sowohl in E. coli wie z.B. auch in Sacharomyces cerevisiae nach entsprechender Umklonierung als Protein synthetisiert und gereinigt werden. Die entsprechenden Prozeduren sind in den Lehrbüchern der Molekularbiologie und Biotechnologie vielfach dargestellt und alle im Prinzip auf die vorliegenden Sequenzen anwendbar. Dem Fachmann sind solche Techniken bekannt. Im folgenden wird die Expression über den Vektor pAC in E. coli (Stamm Top Ten, Firma invitrogen, Groningen, Niederlande) sowie die Reinigung und Charakterisierung der exprimierten Proteine dargestellt.

Die pAC-Derivate, die die zu exprimierenden Sequenzen enthalten, werden zunächst durch Elektroporation in E.coli Top Ten transformiert und die Transformationsmischungen auf Agarplatten (LB-Medium, 50µg/ml Ampicillin) ausgestrichen und über Nacht bei 30°C bebrütet.

Am folgenden Tag werden je 5ml LB-Medium (Zusammensetzung s. (Sambrook et al. 1989); enthaltend: 50µg/ml Ampicillin) mit Bakterien, die von einer einzelnen Kolonie aufgenommen wurden, beimpft. Diese Kultur wird auf einem Inkubationsschüttler bei 300 Umdrehungen pro Minute (rpm) und 37°C über Nacht bebrütet. Am folgenden Kultur wird die Expressionskultur aus dieser über Nacht-Vorkultur mit einer Verdünnung von 1:100 angeimpft und in einem Fernbachkolben bei 300rpm und 37°C solange inkubiert, bis eine optische Dichte (bei 600nm gemessen: OD600) von 0,5 erreicht wird. Dies ist in der Regel nach ca. 2,5h der Fall. Nach Erreichen der gewünschten OD600 wird die Expression durch Zugabe von L-Arabinose (bis zu einer maximalen Konzentration von 0,02%; die optimale Konzentration muss für jeden einzelnen Klon individuell in Vorversuchen ermittelt werden) induziert. Die Expression wird nun bei 26°C, 300rpm und für 4-6h durchgeführt. Das Wachstumsverhalten der Bakterien wird durch Probenahme und Messung der OD600 dokumentiert. Im Anschluss an diese Expressionsphase wird die Kultur 20min. bei 4000g und 4°C zentrifugiert und das Pellet in 2ml TES-Puffer (0.2M Tris-HCl, pH 8.0; 0,5mM EDTA; 0,5M Sucrose) resuspendiert. Zu dieser Suspension werden nunmehr langsam 3ml 1/5 TES-Puffer (1 Volumenteil TES-Puffer plus 4 Volumen Aqua bidest) zugegeben, gemischt und 30min auf Eis inkubiert. Danach wird die Probe in 1,5ml Reaktionsgefäße aliquotiert und bei 10000g 10min bei 4°C zentrifugiert. Der Überstand enthält dann die periplasmatischen Proteine und damit auch das gewünschte Expressionsprodukt.

Zur weiteren Aufarbeitung wird der Überstand über Nacht bei 4°C gegen isotone, phosphatgepufferte Kochsalzlösung (pH 7.4, enthaltend: 10mM Imidazol) dialysiert. Mittels kommerziell erhältlicher Kits zur Nickelaffinitätschromatographie (z.B. Qiagen GmbH, Hilden, Deutschland) kann das gewünschte Protein nunmehr gereinigt werden. Die Reinigung wird mittels Gelelektrophorese kontrolliert und die Proteinkonzentration bestimmt (Harlow and Lane 1988). Molare Konzentrationen wurden aus der der Konzentrationsbestimmung und der aus der Sequenz abgeleiteten Formelmasse bestimmt.

Die gereinigten und quantitativ analysierten Proteine werden anschließend im Fusionsassay unter äquimolaren Bedingungen daraufhin überprüft, ob sie die Fusion menschlicher Trophoblastzellen hemmen können.

Im folgenden' sind die im Beispiel exprimierten und analysierten Klone aufgelistet und die Aminosäuresequenzen, die aus DNA-Sequenzen übersetzt sind, im Alignment dargestellt.

### Fusionsassay, Durchführung und Ergebnisse

Der Nachweis der fusionshemmenden Wirkung wird mit Hilfe der spontan fusionierenden humanen Trophoblastzellinie AC-1M17 geführt. Zellen dieser Zellinie zeigen unmittelbar nach Aussaat in ein neues Kulturgefäß ein initiale Fusionsrate von 20-30%.

Zellen dieser Zellinie werden zunächst zu einer ausreichenden Anzahl vermehrt und dann in zwei Populationen geteilt, die mit unterschiedlichen, kommerziell erhältlichen Fluoreszenzfarbstoffen (DiI und DiO, Färbung nach Angaben des Lieferanten Molecular Probes BV, Leiden, Niederlande) gefärbt werden.

Nachdem die Zellen gründlich gewaschen wurden (5x 10 Minuten), um eventuell anhängende, nicht in die Zellmembran integrierte Farbstoffreste abzuwaschen, werden die beiden Zeflpopulationen gemischt und gemeinsam bei niedriger Zelldichte für 24 Stunden unter Standardkulturbedingungen (5% Kohlendioxid, 38°C, über 90° Luftfeuchte) in Ham's F12 (10% fetales Kälberserum und Antibiotika) inkubiert. Nach dieser Kulturphase werden die Zellen mit Hilfe von Trypsin vom Boden der Kulturflasche abgelöst, suspendiert und im Flow Cytometer bestimmt, wie viele der vorhandenen Zellen durch Fusion zu einer doppelten Fluoreszenz gekommen sind. Figur 1 zeigt ein Beispiel für eine solche Auswertung des Fusionsassays. Figur 2 fasst die für die einzelnen im Beispiel angeführten scFv-Klone gemessenen Hemmraten der Fusion dieser Trophoblastzellen zusammen. Die Figur zeigt ebenfalls die Konzentrationsabhängigkeit des fusionshemmenden Effektes bei den verschiedenen Klonen.

### Literaturliste

Harlow E, Lane D (1988) Antibodies: A laboratory manual. Cold Spring Harbor Laboratory, Cold Spring Harbor, NY
Huppertz B, Tews DS, Kaufmann P (2001) Apoptosis and syncytial fusion in human placental trophoblast and skeletal muscle. Int Rev Cytol 205:215-253
Mi S, Lee X, Li X, Veldman GM, Finnerty H, Racie L, LaVallie E, Tang X-Y, Edouard P, Howes S, Keith JC, McCoy JM (2000) Syncytin is a captive retroviral envelope protein involved in human placental morphogenesis. Nature 403: 785-789
Sambrook J, Fritsch EF, Maniatis T (1989) Molecular cloning :a laboratory manual, 2nd ed. edn. Cold Spring Harbor Laboratory, Cold Spring Harbor, N.Y
Andris-Widhopf J, Rader C, Steinberger P, Fuller R, Barbas III CF, (2000). Methods for the generation of chicken monodonal antibody fragments by phage display. J Immun Meth 242: 159-181

## Patentansprüche

1. Protein, das mit VI oder Vh des Huhns zu mindestens 80 % homolog ist und eine synzytiale Fusion von Säugetier-Trophoblasten hemmt.
wobei VI die nachfolgende Sequenz aufweist: wobei
X⁰ = eine unsubstituierte, mono oder disubstituierte Aminogruppe oder eine oder mehrere Aminesäuren bedeutet,
X¹ = P oder L,
X² = G oder E,
X³ = K oder E,
X⁴ = I oder L,
X⁵ = G oder S,
X⁶ = S oder G,
X⁷ = G oder R oder Y,
X⁸ = S oder R oder Y oder eine Deletion,
X⁹ = W oder S oder eine Deletion,
X¹⁰ = S oder K oder Y oder eine Deletion,
X¹¹ = Y oder F,
X¹² = S oder A oder eine Deletion,
X¹³ = A oder T oder eine Deletion,
X¹⁴ = V oder D,
X¹⁵ = V oder L,
X¹⁶ = N oder V oder S oder D oder E,
X¹⁷ = N oder S,
X¹⁸ = N oder T oder D oder K,
X¹⁹ = K oder N,
X²⁰ = D oder N,
X²¹ = K oder V,
X²² = T oder A,
X²³ = A oder H oder N,
X²⁴ = D oder E,
X²⁵ = Y oder F,
X²⁶ = N oder 5 oder G,
X²⁷ = R oder Y oder G oder eine Deletion,
X²⁸ = D oder eine Deletion,
X²⁹ = W oder eine Deletion,
X³⁰ = D oder K oder A oder eine Deletion,
X³¹ = S oder T oder D,
X³² = S oder A oder G,
X³³ = A oder G oder eine Deletion,
X³⁴ = G oder R oder eine Deletion,
X³⁵ = Y oder N oder eine Detetion,
X³⁶ = V oder A oder T,
X³⁷ = G oder A oder N,
X³⁸ = I oder A oder M und
X³⁹ = eine Carboxylgruppe, ein Carboxylderivat oder eine oder mehrere Aminosäuren bedeutet
und wobei konservative Austausche jeder beliebigen Aminosäure untereinander zulässig sind, sofern die Hemmung der synzytialen Fusion von Säugetier-Trophoblasten erhalten bleibt.
wobei Vh die nachfolgende Sequenz aufweist: wobei
X⁰ = eine unsubstituierte, mono oder disubstituierte Aminogruppe oder eine oder mehrere Aminosäuren bedeutet,
X¹ = A oder S oder T,
X² = G oder R,
X³ = G oder A oder T,
X⁴ = A oder G,
X⁵ = S oder T oder D,
X⁶ = F oder M,
X⁷ = S oder T,
X⁸ = S oder D oder N oder eine Deletion,
X⁹ = G oder Q oder A oder T,
X¹⁰ = N oder A oder Q oder G,
X¹¹ = I oder V,
X¹² = F oder W oder Y,
X¹³ A oder G,
X¹⁴ = G oder A oder R,
X¹⁵ = I oder M,
X¹⁶ = 5 oder N oder G,
X¹⁷ = S oder R oder N,
X¹⁸ = G oder eine Deletion,
X¹⁹ = F oder T oder S oder N,
X²⁰ = G oder S oder A,
X²¹ = N oder S oder R,
X²² = R oder S oder Y,
X²³ = G oder A oder N oder Y,
X²⁴ = G oder A,
X²⁵ = S oder A oder P,
X²⁶ = K oder Q,
X²⁷ = R oder L,
X²⁸ = A oder C,
X²⁹ = T oder H,
X³⁰ = I oder H,
X³¹ = S oder L,
X³² = R oder E,
X³³ = D oder G,
X³⁴ = N oder Q oder R oder D oder K,
X³⁵ = G oder R oder W,
X³⁶ = Q oder A,
X³⁷ = S oder E,
X³⁸ = T oder H,
X³⁹ = V oder S,
X⁴⁰ = R oder E,
X⁴¹ = L oder A,
X⁴² = Q oder A,
X⁴³ = L oder A,
X⁴⁴ = N oder E oder S,
X⁴⁵ = N oder Q,
X⁴⁶ = L oder P,
X⁴⁷ = R oder Q,
X⁴⁸ = A oder G,
X⁴⁹ = E oder G,
X⁵⁰ = D oder H,
X⁵¹ = T oder R oder L,
X⁵² = G oder P oder eine Deletion,
X⁵³ = T oder P oder eine Deletion,
X⁵⁴ = Y oder T oder eine Deletion,
X⁵⁵ = Y oder S oder eine Deletion,
X⁵⁶ = C oder A oder eine Deletion,
X⁵⁷ = A oder P oder eine Deletion,
X⁵⁸ = K oder eine Deletion ist,
X⁵⁹ = eine carboxylgruppe, ein Carboxylderivat oder eine oder mehrere Aminosäuren bedeutet
und wobei konservative Austausche jeder beliebigen Aminosäure untereinander zulässig sind, sofern die Hemmung der synzytialen Fusion von Trophoblasten-Zellen erhalten bleibt,
wobei die Proteine mit den Sequenzen VI und Vh miteinander über einen Linker verbunden sind.

2. Protein nach Anspruch 1, wobei der Linker eine Peptidkette aus mindestens 5 beliebigen Aminosäuren ist.

3. Protein nach einem der Ansprüche 1 oder 2, wobei D-Aminosäuren in der Sequenz enthalten sind.

4. Protein nach mindestens einem der Ansprüche 1 bis 3 mit den Aminosäuresequenzen Seq ID Nr. 1 bis 10.

5. Protein mit der Sequenz Seq ID Nr. 11 (Konsensussequenz).

6. Nucleinsäure, insbesondere DNA oder RNA, codierend für ein Protein nach einem der Ansprüche 1 bis 5.

7. Plasmid oder Vektor, enthaltend eine Nucleinsäure nach Anspruch 6.

8. Arzneimittel enthaltend mindestens eines der Proteine gemäß einem der Ansprüche 1 bis 5 oder mindestens eine Nucleinsäure nach Anspruch 6.

9. Verwendung mindestens eines der Proteine gemäß einem der Ansprüche 1 bis 5 oder mindestens einer Nucleinsäure nach Anspruch 6 zur Herstellung eines Arzneimittels zur Kontrazeption bei Säugern.

## Claims

1. A protein which is homologous with chicken Vl or Vh to at least 80% and inhibits the syncytial fusion of mammal trophoblasts, wherein Vl has the following sequence: wherein
X⁰ = an unsubstituted, mono- or disubstituted amino group or one or more amino acids;
X¹ = P or L;
X² = G or E;
X³ = K or E;
X⁴ = I or L;
X⁵ = G or S;
X⁶ = S or G;
X⁷ = G or R or Y;
X⁸ = S or R or Y or a deletion;
X⁹ = W or S or a deletion;
X¹⁰ = S or K or Y or a deletion;
X¹¹ = Y or F;
X¹² = S or A or a deletion;
X¹³ = A or T or a deletion;
X¹⁴ = V or D;
X¹⁵ = VorL;
X¹⁶ = NorVorSorDorE;
X¹⁷ = N or S;
X¹⁸ = N or T or D or K;
X¹⁹= KorN;
X²⁰ = D or N;
X²¹ = K or V;
X²² = T or A;
X²³ = AorHorN;
X²⁴ = D or E;
X²⁵ = Y or F;
X²⁶ = N or S or G;
X²⁷ = R or Y or G or a deletion;
X²⁸ = D or a deletion;
X²⁹ = W or a deletion;
X³⁰ = D or K or A or a deletion;
X³¹ = S or T or D;
X³² = S or A or G;
X³³ = A or G or a deletion;
X³⁴ = G or R or a deletion;
X³⁵ = Y or N or a deletion;
X³⁶ = V or A or T;
X³⁷ = G or A or N;
X³⁸ = I or A or M; and
X³⁹ = a carboxy group, a carboxy derivative or one or more amino acids;
and wherein mutual conservative exchanges of any amino acids are admissible as long as the inhibition of the syncytial fusion of mammal trophoblasts is retained;
wherein Vh has the following sequence: wherein
X⁰ = an unsubstituted, mono- or disubstituted amino group or one or more amino acids;
X¹ = A or S or T;
X² = G or R;
X³ = G or A or T;
X⁴ = A or G;
X⁵ = S or T or D;
X⁶ = F or M;
X⁷ = S or T;
X⁸ = S or D or N or a deletion;
X⁹ = G or Q or A or T;
X¹⁰ = N or A or Q or G;
X¹¹ = I or V;
X¹² = F or W or Y;
X¹³ = A or G;
X¹⁴ = G or A or R;
X¹⁵ = I or M;
X¹⁶ = S or N or G;
X¹⁷ = S or R or N;
X¹⁸ = G or a deletion;
X¹⁹ = F or T or S or N;
X²⁰ = G or S or A;
X²¹ = N or S or R;
X²² = R or S or Y;
X²³ = G or A or N or Y;
X²⁴ = G or A;
X²⁵ = S or A or P;
X²⁶ = K or Q;
X²⁷ = R or L;
X²⁸ = A or C;
X²⁹ = T or H;
X³⁰ = I or H;
X³¹ = S or L;
X³² = R or E;
X³³ = D or G;
X³⁴ = N or Q or R or D or K;
X³⁵ = G or R or W;
X³⁶ = Q or A;
X³⁷ = S or E;
X³⁸ = T or H;
X³⁹ = V or S;
X⁴⁰ = R or E;
X⁴¹ = L or A;
X⁴² = Q or A;
X⁴³ = L or A;
X⁴⁴ = N or E or S;
X⁴⁵ = N or Q;
X⁴⁶ = L or P;
X⁴⁷ = R or Q;
X⁴⁸ = A or G;
X⁴⁹ = E or G;
X⁵⁰ = D or H;
X⁵¹ = T or R or L;
X⁵² = G or P or a deletion;
X⁵³ = T or P or a deletion;
X⁵⁴ = Y or T or a deletion;
X⁵⁵ = Y or S or a deletion;
X⁵⁶ = C or A or a deletion;
X⁵⁷ = A or P or a deletion;
X⁵⁸ = K or a deletion;
X⁵⁹ = a carboxy group, a carboxy derivative or one or more amino acids;
and wherein mutual conservative exchanges of any amino acids are admissible as long as the inhibition of the syncytial fusion of trophoblast cells is retained;
wherein the proteins with the sequences VI and Vh are interconnected through a linker.

2. The protein according to claim 1, wherein said linker is a peptide chain which consists of at least any five amino acids.

3. The protein according to either of claims 1 or 2, wherein D-amino acids are contained in the sequence.

4. The protein according to at least one of claims 1 to 3 with the amino acid sequences SEQ ID Nos. 1 to 10.

5. A protein having the sequence SEQ ID No. 11 (consensus sequence).

6. A nucleic acid, especially DNA or RNA, coding for a protein according to any of claims 1 to 5.

7. A plasmid or vector containing a nucleic acid according to claim 6.

8. A medicament containing at least one of the proteins according to any of claims 1 to 5 or at least one nucleic acid according to claim 6.

9. Use of at least one of the proteins according to any of claims 1 to 5 or at least one nucleic acid according to claim 6 for preparing a medicament for contraception in mammals.

## Revendications

1. Protéine qui est une homologue à au moins 80 % de la région VI ou Vh du poulet et inhibe une fusion syncytiale des trophoblastes de mammifères,
dans laquelle VI a la séquence suivante : dans laquelle
X⁰ représente un groupe amino non substitué, mono- ou disubstitué ou un ou plusieurs acides aminés,
X¹ = P ou L,
X² = G ou E,
X³ = K ou E,
X⁴ = I ou L,
X⁵ = G ou S,
X⁶ = S ou G,
X⁷ = G ou R ou Y,
X⁸ = S ou R ou Y ou une délétion,
X⁹ = W ou S ou une délétion,
X¹⁰ = S ou K ou Y ou une délétion,
X¹¹ = Y ou F,
X¹² = S ou A ou une délétion,
X¹³ = A ou T ou une délétion,
X¹⁴ = V ou D,
X¹⁵ = V ou L,
X¹⁶ = N ou V ou S ou D ou E,
X¹⁷ = N ou S,
X¹⁸ = N ou T ou D ou K,
X¹⁹ = K ou N,
X²⁰ = D ou N,
X²¹ = K ou V,
X²² = T ou A,
X²³ = A ou H ou N,
X²⁴ = D ou E,
X²⁵ = Y ou F,
X²⁶ = N ou S ou G,
X²⁷ = R ou Y ou G ou une délétion,
X²⁸ = D ou une délétion,
X²⁹ = W ou une délétion,
X³⁰ = D ou K ou A ou une délétion,
X³¹ = S ou T ou D,
X³² = S ou A ou G,
X³³ = A ou G ou une délétion,
X³⁴ = G ou R ou une délétion,
X³⁵ = Y ou N ou une délétion,
X³⁶ = V ou A ou T,
X³⁷ = G ou A ou N,
X³⁸ = I ou A ou M et
X³⁹ représente un groupe carboxyle, un dérivé de carboxyle ou un ou plusieurs acides aminés,
et dans laquelle des substitutions conservatives mutuelles de n'importe quel acide aminé par un autre sont permises, dans la mesure où l'inhibition de la fusion syncytiale des trophoblastes de mammifères est maintenue,
dans laquelle Vh a la séquence suivante : dans laquelle
X⁰ représente un groupe amino non substitué, mono- ou disubstitué ou un ou plusieurs acides aminés,
X¹ = A ou S ou T,
X² = G ou R,
X³ = G ou A ou T,
X⁴ = A ou G,
X⁵ = S ou T ou D,
X⁶ = F ou M,
X⁷ = S ou T,
X⁸ = S ou D ou N ou une délétion,
X⁹ = G ou Q ou A ou T,
X¹⁰ = N ou A ou Q ou G,
X¹¹ = I ou V,
X¹² = F ou W ou Y,
X¹³ = A ou G,
X¹⁴ = G ou A ou R,
X¹⁵ = I ou M,
X¹⁶ = S ou N ou G,
X¹⁷ = S ou R ou N,
X¹⁸ = G ou une délétion,
X¹⁹ = F ou T ou S ou N,
X²⁰ = G ou S ou A,
X²¹ = N ou S ou R,
X²² = R ou S ou Y,
X²³ = G ou A ou N ou Y,
X²⁴ = G ou A,
X²⁵ = S ou A ou P,
X²⁶ = K ou Q,
X²⁷ = R ou L,
X²⁸ = A ou C,
X²⁹ = T ou H,
X³⁰ = I ou H,
X³¹ = S ou L,
X³² = R ou E,
X³³ = D ou G,
X³⁴ = N ou Q ou R ou D ou K,
X³⁵ = G ou R ou W,
X³⁶ = Q ou A,
X³⁷ = S ou E,
X³⁸ = T ou H,
X³⁹ = V ou S,
X⁴⁰ = R ou E,
X⁴¹ = L ou A,
X⁴² = Q ou A,
X⁴³ = L ou A,
X⁴⁴ = N ou E ou S,
X⁴⁵ = N ou Q,
X⁴⁶ = L ou P,
X⁴⁷ = R ou Q,
X⁴⁸ = A ou G,
X⁴⁹ = E ou G,
X⁵⁰ = D ou H,
X⁵¹ = T ou R ou L,
X⁵² = G ou P ou une délétion,
X⁵³ = T ou P ou une délétion,
X⁵⁴ = Y ou T ou une délétion,
X⁵⁵ = Y ou S ou une délétion,
X⁵⁶ = C ou A ou une délétion,
X⁵⁷ = A ou P ou une délétion,
X⁵⁸ = K ou est une délétion,
X⁵⁹ représente un groupe carboxyle, un dérivé de carboxyle ou un ou plusieurs acides aminés,
et dans laquelle des substitutions conservatives mutuelles de n'importe quel acide aminé par un autre sont permises, dans la mesure où l'inhibition de la fusion syncytiale des cellules de trophoblastes de mammifères est maintenue,
moyennant quoi les protéines ayant les séquences VI et Vh sont associées ensemble par l'intermédiaire d'un espaceur.

2. Protéine selon la revendication 1, dans laquelle l'espaceur est une chaîne peptidique comportant au moins 5 acides aminés au choix.

3. Protéine selon une des revendications 1 ou 2, dans laquelle la séquence contient des acides aminés de forme D.

4. Protéine selon au moins une des revendications 1 à 3, ayant une des séquences d'acides aminés ayant pour identificatifs les Seq. ID N° 1 à 10.

5. Protéine ayant la séquence d'identification Seq ID N° 11 (séquence de consensus).

6. Acide nucléique, notamment ADN ou ARN, codant pour une protéine selon une des revendications 1 à 5.

7. Plasmide ou vecteur, contenant un acide nucléique selon la revendication 6.

8. Médicament contenant au moins une des protéines selon une des revendications 1 à 5 ou au moins un acide nucléique selon la revendication 6.

9. Utilisation d'au moins une des protéines selon une des revendications 1 à 5 ou d'au moins un acide nucléique selon la revendication 6 pour préparer un médicament destiné à la contraception des mammifères.


### SEQUENCE LISTING

<110> Aplagen GmbH
<120> Immunologische Bindungsmoleküle, die die synzytiale Fusion von Trophoblastzellen hemmen
<130> 032705ep
<140> EP02782453.1
   <141> 2002-07-02
<160> 11
<170> Patentln Ver. 2.1
<210> 1
   <211> 209
   <212> PRT
   <213> Artificial Sequence
<220>
   <223> Description of Artificial Sequence: Hybridsequenz
<400> 1
   Ala Leu Thr Gln Pro Ser Ser Val Ser Ala Asn Leu Gly Gly Thr Val
   1 5 10 15
   Glu Ile Thr Cys Ser Gly Gly Gly Arg Ser Trp Tyr Gly Trp Tyr Gln
   20 25 30
   Gln Lys Ser Pro Gly Ser Ala Pro Val Thr Val Ile Tyr Asn Asn Asn
   35 40 45
   Lys Arg Pro Ser Asp Ile Pro Ser Arg Phe Ser Gly Ser Lys Ser Gly
   50 55 60
   Ser Thr Ala Thr Leu Thr Ile Thr Gly Val Gln Ala Asp Asp Glu Ala
   65 70 75 80
   Val Tyr Tyr Cys Gly Ser Ser Ala Gly Arg Asn Ala Ala Ala Phe Gly
   85 90 95
   Ala Gly Thr Thr Leu Thr Val Leu Gly Gln Ser Ser Arg Ser Ser Ala
   100 105 110
   Val Thr Leu Asp Glu Ser Gly Gly Gly Leu Gln Thr Pro Gly Gly Gly
   115 120 125
   Leu Ser Leu Val Cys Lys Ala Ser Gly Phe Asp Phe Ser Asn Tyr Gln
   130 135 140
   Met Asn Trp Ile Arg Gln Ala Pro Gly Lys Gly Leu Glu Phe Val Ala
   145 150 155 160
   Ala Ile Asn Ser Phe Gly Asn Arg Thr Gly Tyr Ala Ala Ala Val Lys
   165 170 175
   Gly Arg Ala Thr Ile Ser Arg Asp Asp Gly Gln Ser Thr Val Arg Leu
   180 185 190
   Gln Leu Ser Asn Leu Arg Ala Glu Asp Thr Gly Thr Tyr Tyr Cys Ala
   195 200 205
   Lys
<210> 2
   <2i 1> 210
   <212> PRT
   <213> Artificial Sequence
<220>
   <223> Description of Artificial Sequence:
   Konsensussequenz
<400> 2
<210> 3
   <211> 215
   <212> PRT
   <213> Artificial Sequence
<220>
   <223> Description of Artificial Sequence: Hybridsequenz
<400> 3
   Ala Leu Thr Gln Pro Ser Ser Val Ser Ala Asn Pro Gly Gly Thr Val
   1 5 10 15
   Lys Ile Thr Cys Ser Gly Gly Ser Gly Ser Trp Ser Tyr Gly Trp Tyr
   20 25 30
   Gln Gln Lys Ser Pro Gly Ser Ala Pro Val Thr Val Ile Tyr Asn Asn
   35 40 45
   Asn Lys Arg Pro Ser Asp Ile Pro Ser Arg Phe Ser Gly Ser Lys Ser
   50 55 60
   Gly Ser Thr Ala Thr Leu Thr Ile Thr Gly Phe Gln Ala Asp Asp Glu
   65 70 75 80
   Ala Val Tyr Tyr Cys Gly Asn Arg Asp Trp Asp Ser Ser Ala Gly Tyr
   85 90 95
   Val Gly Ile Phe Gly Ala Gly Thr Thr Leu Thr Val Leu Gly Xaa Xaa
   100 105 110
   Xaa Xaa Xaa Xaa Ala Val Thr Leu Asp Glu Ser Gly Gly Gly Leu Gln
   115 120 125
   Thr Pro Gly Gly Gly Leu Ser Leu Val Cys Lys Ala Ser Gly Phe Ser
   130 135 140
   Phe Ser Ser Tyr Gln Met Asn Trp Ile Arg Gln Ala Pro Gly Lys Gly
   145 150 155 160
   Leu Glu Phe Val Ala Gly Ile Ser Ser Gly Phe Gly Asn Arg Thr Gly
   165 170 175
   Tyr Gly Ser Ala Val Lys Gly Arg Ala Thr Ile Ser Arg Asp Asn Gly
   180 185 190
   Gln Ser Thr Val Arg Leu Gln Leu Asn Asn Leu Arg Ala Glu Asp Thr
   195 200 205
   Gly Thr Tyr Tyr Cys Ala Lys
   210 215
<210> 4
   <211> 211
   <212> PRT
   <213> Artificial Sequence
<220>
   <223> Description of Artificial Sequence: Hybridsequenz
<400> 4
   Ala Leu Thr Gln Pro Ser Ser Val Ser Ala Asn Pro Gly Glu Thr Val
   1 5 10 15
   Lys Ile Thr Cys Ser Gly Gly Gly Ser Tyr Tyr Gly Trp Tyr Gln Gln
   20 25 30
   Lys Ala Pro Gly Ser Ala Pro Val Thr Val Ile Tyr Asp Asn Thr Asn
   35 40 45
   Arg Pro Ser Asn Ile Pro Ser Arg Phe Ser Gly Ser Lys Ser Gly Ser
   50 55 60
   Thr His Thr Leu Thr Ile Thr Gly Val Gln Ala Asp Asp Glu Ala Val
   65 70 75 80
   Tyr Phe Cys Gly Ser Tyr Asp Ser Ser Ala Gly Tyr Val Gly Ile Phe
   85 90 95
   Gly Ala Gly Thr Thr Leu Thr Val Leu Gly Gln Ser Ser Arg Ser Ser
   100 105 110
   Ala Val Thr Leu Asp Glu Ser Gly Gly Gly Leu Gln Thr Pro Gly Gly
   115 120 125
   Gly Leu Ser Leu Val Cys Lys Ala Ser Gly Phe Thr Phe Ser Asp Tyr
   130 135 140
   Gly Met Gly Trp Val Arg Gln Ala Pro Gly Lys Gly Leu Glu Phe Val
   145 150 155 160
   Ala Arg Ile Ser Ser Gly Ser Gly Ser Ser Thr Gly Tyr Gly Ser Ala
   165 170 175
   Val Lys Gly Arg Ala Thr Ile Ser Arg Asp Lys Gly Gln Ser Thr Val
   180 185 190
   Arg Leu Gln Leu Asn Asn Leu Arg Ala Glu Asp Thr Gly Thr Tyr Tyr
   195 200 205
   Cys Ala Lys
   210
<210> 5
   <211> 207
   <212> PRT
   <213> Artificial Sequence
<220>
   <223> Description of Artificial Sequence: Hybridsequenz
<400> 5
   Ala Leu Thr Gln Pro Ser Ser Val Ser Ala Asn Pro Gly Glu Thr Val
   1 5 10 15
   Lys Ile Thr Cys Ser Gly Gly Gly Ser Ser Tyr Gly Trp Tyr Gln Gln
   20 25 30
   Lys Ser Pro Gly Ser Thr Pro Val Thr Val Ile Tyr Asn Asn Lys Asn
   35 40 45
   Arg Pro Ser Asp Ile Pro Ser Arg Phe Ser Gly Ser Lys Ser Gly Ser
   50 55 60
   Ala Asn Thr Leu Thr Ile Thr Gly Val Gln Ala Asp Asp Glu Ala Val
   65 70 75 80
   Tyr Phe Cys Gly Ser Arg Ala Asp Asn Val Gly Ile Phe Gly Ala Gly
   85 90 95
   Thr Thr Leu Thr Val Leu Gly Gln Ser Ser Arg Ser Ser Thr Val Thr
   100 105 110
   Leu Asp Glu Ser Gly Gly Gly Leu Gln Thr Pro Gly Arg Thr Leu Ser
   115 120 125
   Leu Val Cys Lys Ala Ser Gly Phe Ser Phe Ser Ser Tyr Gly Met Ala
   130 135 140
   Trp Val Arg Gln Ala Pro Gly Lys Gly Leu Glu Phe Val Ala Gly Ile
   145 150 155 160
   Ser Asn Thr Gly Ser Ser Thr Gly Tyr Gly Ala Ala Val Lys Gly Arg
   165 170 175
   Ala Thr Ile Ser Arg Asp Asn Gly Gln Ser Thr Val Arg Leu Gln Leu
   180 185 190
   Asn Asn Leu Arg Ala Glu Asp Thr Gly Thr Tyr Tyr Cys Ala Lys
   195 200 205
<210> 6
   <211> 203
   <212> PRT
   <213> Artificial Sequence
<220>
   <223> Description of Artificial Sequence: Hybridsequenz
<400> 6
   Ala Leu Thr Gln Pro Ser Ser Val Ser Ala Asn Pro Gly Glu Thr Val
   1 5 10 15
   Lys Leu Thr Cys Ser Gly Gly Ser Tyr Ser Ser Tyr Tyr Gly Trp Tyr
   20 25 30
   Gln Gln Lys Ser Pro Gly Ser Ala Pro Val Thr Leu Ile Tyr Glu Ser
   35 40 45
   Asn Lys Arg Pro Ser Asn Ile Pro Ser Arg Phe Ser Gly Ser Lys Ser
   50 55 60
   Gly Ser Thr Ala Thr Leu Thr Ile Thr Gly Val Gln Ala Glu Asp Glu
   65 70 75 80
   Ala Val Tyr Tyr Cys Gly Gly Arg Asp Ser Ser Tyr Val Asn Ile Phe
   85 90 95
   Gly Ala Gly Thr Thr Leu Thr Val Leu Gly Gln Ser Ser Arg Ser Ser
   100 105 110
   Ala Val Thr Leu Asp Glu Ser Gly Gly Gly Leu Gln Thr Pro Gly Gly
   115 120 125
   Gly Leu Ser Leu Val Cys Lys Ala Ser Gly Phe Ser Phe Ser Ser Tyr
   130 135 140
   Thr Met Gln Trp Val Arg Gln Ala Pro Gly Lys Gly Leu Glu Tyr Val
   145 150 155 160
   Ala Gly Ile Ser Ser Asn Ser Arg Tyr Thr Tyr Tyr Gly Ser Ala Val
   165 170 175
   Lys Gly Leu Cys His His Leu Glu Gly Gln Arg Ala Glu His Ser Glu
   180 185 190
   Ala Ala Ala Glu Gln Pro Gln Gly Gly His Arg
   195 200
<210> 7
   <211> 211
   <212> PRT
   <213> Artificial Sequence
<220>
   <223> Description of Artificial Sequence: Hybridsequenz
<400> 7
   Ala Leu Thr Gln Pro Ser Ser Val Ser Ala Asn Pro Gly Glu Thr Val
   1 5 10 15
   Lys Ile Thr Cys Ser Gly Gly Gly Ser Tyr Tyr Gly Trp Tyr Gln Gln
   20 25 30
   Lys Ala Pro Gly Ser Ala Pro Val Thr Val Ile Tyr Asp Asn Thr Asn
   35 40 45
   Arg Pro Ser Asn Ile Pro Ser Arg Phe Ser Gly Ser Lys Ser Gly Ser
   50 55 60
   Thr His Thr Leu Thr Ile Thr Gly Val Gln Ala Asp Asp Glu Ala Val
   65 70 75 80
   Tyr Phe Cys Gly Ser Tyr Asp Ser Ser Ala Gly Tyr Val Gly Ile Phe
   85 90 95
   Gly Ala Gly Thr Thr Leu Thr Val Leu Gly Gln Ser Ser Arg Ser Ser
   100 105 110
   Ala Val Thr Leu Asp Glu Ser Gly Gly Gly Leu Gln Thr Pro Gly Gly
   115 120 125
   Gly Leu Ser Leu Val Cys Lys Ala Ser Gly Phe Thr Phe Ser Asp Tyr
   130 135 140
   Gly Met Gly Trp Val Arg Gln Ala Pro Gly Lys Gly Leu Glu Phe Val
   145 150 155 160
   Ala Arg Ile Ser Ser Gly Ser Gly Ser Ser Thr Gly Tyr Gly Ser Ala
   165 170 175
   Val Lys Gly Arg Ala Thr Ile Ser Arg Asp Lys Gly Gln Ser Thr Val
   180 185 190
   Arg Leu Gln Leu Asn Asn Leu Arg Ala Glu Asp Thr Gly Thr Tyr Tyr
   195 200 205
   Cys Ala Lys
   210
<210> 8
   <211> 203
   <212> PRT
   <213> Artificial Sequence
<220>
   <223> Description of Artificial Sequence: Hybridsequenz
<400> 8
   Ala Leu Thr Gln Pro Ser Ser Val Ser Ala Asn Pro Gly Gly Thr Val
   1 5 10 15
   Lys Ile Thr Cys Ser Gly Ser Ser Gly Ser Tyr Gly Trp Tyr Gln Gln
   20 25 30
   Lys Ser Pro Gly Ser Ala Pro Val Thr Val Ile Tyr Asn Asn Asn Lys
   35 40 45
   Arg Pro Ser Asp Ile Pro Ser Arg Phe Ser Gly Ser Lys Ser Gly Ser
   50 55 60
   Thr Ala Thr Leu Thr Ile Thr Gly Val Gln Ala Glu Asp Glu Ala Val
   65 70 75 80
   Tyr Phe Cys Gly Ser Arg Asp Ser Ser Ala Gly Tyr Val Gly Ile Phe
   85 90 95
   Gly Ala Gly Thr Thr Leu Thr Val Leu Gly Gln Ser Ser Arg Ser Ser
   100 105 110
   Ser Val Thr Leu Asp Glu Ser Gly Gly Gly Leu Gln Thr Pro Gly Gly
   115 120 125
   Ala Leu Ser Leu Val Cys Lys Ala Ser Gly Phe Ser Met Ser Ser Tyr
   130 135 140
   Gln Met Asn Trp Ile Arg Gln Ala Pro Gly Lys Gly Leu Glu Trp Val
   145 150 155 160
   Ala Gly Met Asn Arg Phe Ala Asn Arg Thr Asn Tyr Gly Ser Ala Val
   165 170 175
   Lys Gly Arg Cys His His Leu Glu Gly Arg Trp Ala Glu His Ser Glu
   180 185 190
   Ala Ala Ala Glu Gln Pro Gln Gly Gly His Arg
   195 200
<210> 9
   <211> 210
   <212> PRT
   <213> Artificial Sequence
<220>
   <223> Description of Artificial Sequence: Hybridsequenz
<400> 9
   Ala Leu Thr Gln Pro Ser Ser Val Ser Ala Asn Leu Gly Gly Thr Val
   1 5 10 15
   Lys Ile Thr Cys Ser Gly Gly Ser Ser Arg Lys Tyr Gly Trp Tyr Gln
   20 25 30
   Gln Lys Ser Pro Gly Ser Ala Pro Asp Thr Val Ile Tyr Ser Asn Asp
   35 40 45
   Asn Arg Pro Ser Asp Ile Pro Ser Arg Phe Ser Gly Ser Val Ser Gly
   50 55 60
   Ser Thr Asn Thr Leu Thr Ile Thr Gly Val Gln Ala Glu Asp Glu Ala
   65 70 75 80
   Val Tyr Phe Cys Gly Ser Gly Asp Trp Lys Thr Gly Thr Gly Met Phe
   85 90 95
   Gly Ala Gly Thr Thr Leu Thr Val Leu Gly Gln Ser Ser Arg Ser Ser
   100 105 110
   Ala Val Thr Leu Asp Glu Ser Gly Gly Gly Leu Gln Thr Pro Gly Gly
   115 120 125
   Thr Leu Ser Leu Val Cys Lys Ala Ser Gly Phe Thr Phe Ser Ser Tyr
   130 135 140
   Ala Met Asn Trp Val Arg Gln Ala Pro Gly Lys Gly Leu Glu Phe Val
   145 150 155 160
   Ala Gly Ile Gly Asn Thr Gly Arg Tyr Thr Gly Tyr Gly Pro Ala Val
   165 170 175
   Gln Gly Arg Ala Thr Ile Ser Arg Asp Asn Gly Gln Ser Thr Val Arg
   180 185 190
   Leu Gln Ala Glu Gln Pro Gln Gly Gly His Leu Pro Pro Thr Ser Ala
   195 200 205
   Pro Lys
   210
<210> 10
   <211> 209
   <212> PRT
   <213> Artificial Sequence
<220>
   <223> Description of Artificial Sequence: Hybridsequenz
<400> 10
   Ala Leu Thr Gln Pro Ser Ser Val Ser Ala Asn Leu Gly Gly Thr Val
   1 5 10 15
   Glu Ile Thr Cys Ser Gly Gly Gly Arg Ser Trp Tyr Gly Trp Tyr Gln
   20 25 30
   Gln Lys Ser Pro Gly Ser Ala Pro Val Thr Val Ile Tyr Asn Asn Asn
   35 40 45
   Lys Arg Pro Ser Asp Ile Pro Ser Arg Phe Ser Gly Ser Lys Ser Gly
   50 55 60
   Ser Thr Ala Thr Leu Thr Ile Thr Gly Val Gln Ala Asp Asp Glu Ala
   65 70 75 80
   Val Tyr Tyr Cys Gly Ser Ser Ala Gly Arg Asn Ala Ala Ala Phe Gly
   85 90 95
   Ala Gly Thr Thr Leu Thr Val Leu Gly Gln Ser Ser Arg Ser Ser Ala
   100 105 110
   Val Thr Leu Asp Glu Ser Gly Gly Gly Leu Gln Thr Pro Gly Gly Gly
   115 120 125
   Leu Ser Leu Val Cys Lys Ala Ser Gly Phe Asp Phe Ser Asn Tyr Gln
   130 135 140
   Met Asn Trp Ile Arg Gln Ala Pro Gly Lys Gly Leu Glu Phe Val Ala
   145 150 155 160
   Ala Ile Asn Ser Phe Gly Asn Arg Thr Gly Tyr Ala Ala Ala Val Lys
   165 170 175
   Gly Arg Ala Thr Ile Ser Arg Asp Asp Gly Gln Ser Thr Val Arg Leu
   180 185 190
   Gln Leu Ser Asn Leu Arg Ala Glu Asp Thr Gly Thr Tyr Tyr Cys Ala
   195 200 205
   Lys
<210> 11
   <211> 207
   <212> PRT
   <213> Artificial Sequence
<220>
   <223> Description of Artificial Sequence:
   Konsensussequenz
<400> 11
   Ala Leu Thr Gln Pro Ser Ser Val Ser Ala Asn Leu Gly Gly Thr Val
   1 5 10 15
   Glu Ile Thr Cys Ser Gly Gly Ser Gly Ser Tyr Gly Trp Phe Gln Gln
   20 25 30
   Lys Ser Pro Gly Ser Ala Pro Val Thr Val Ile Tyr Val Asn Thr Asn
   35 40 45
   Arg Pro Ser Asp Ile Pro Ser Arg Phe Ser Gly Ser Lys Ser Gly Ser
   50 55 60
   Thr Ala Thr Leu Thr Ile Thr Gly Val Gln Ala Asp Asp Glu Ala Val
   65 70 75 80
   Tyr Tyr Cys Gly Asn Ser Ala Gly Arg Asn Ala Ala Ala Phe Gly Ala
   85 90 95
   Gly Thr Thr Leu Thr Val Leu Gly Gln Ser Ser Arg Ser Ser Ala Val
   100 105 110
   Thr Leu Asp Glu Ser Gly Gly Gly Leu Gln Thr Pro Gly Gly Gly Leu
   115 120 125
   Ser Leu Val Cys Lys Gly Ser Gly Phe Ser Phe Thr Tyr Gln Met Asn
   130 135 140
   Trp Ile Arg Gln Ala Pro Gly Lys Gly Leu Glu Phe Val Gly Ala Ile
   145 150 155 160
   Ser Arg Phe Gly Asn Ser Thr Ala Tyr Gly Ser Ala Val Lys Gly Arg
   165 170 175
   Ala Thr Ile Ser Arg Asp Asn Gly Gln Ser Thr Val Arg Leu Gln Leu
   180 185 190
   Asn Asn Leu Arg Ala Glu Asp Thr Ala Thr Tyr Phe Cys Ala Lys
   195 200 205